# EUROPEAN PATENT SPECIFICATION

(11) **EP 1 392 240 B1**
(45) Date of publication and mention of the grant of the patent: **03.08.2011**
(21) Application number: 02727757.3
(22) Date of filing: 27.05.2002
(51) Int. Cl.: A61K 9/00

(54) **FORMULATION COMPRISING MELT GRANULES OF NSAIDS AND ORGANIC ACIDS**
FORMULIERUNG ENTHALTEND SCHMELZGRANULIERTE NSAIDS UND ORGANISCHE SÄUREN
FORMULATION CONTENANT DES GRANULES FONDUS DE NSAID ET DES ACIDES ORGANIQUES

(30) Priority: 07.06.2001 GB 0113843
(43) Date of publication of application: 03.03.2004
(73) Proprietor: Reckitt Benckiser Healthcare (UK) Limited, Slough, Berkshire SL1 3UH (GB)
(72) Inventor: PRICE, Ian Ashley, The Boots Company PLC, Nottingham NG2 3AA (GB); SHERRY, Robert Arthur, The Boots Company PLC, Nottingham NG2 3AA (GB); HIGTON, Frederick Raymond, The Boots Company PLC, Nottingham NG2 5EB (GB); WRIGHT, Nicola Wendy, The Boots Company PLC, Nottingham NG2 3AA (GB); BARRETT, David Michael, The Boots Company PLC, Nottingham NG2 3AA (GB)
(74) Representative: O'Brien, Niall James
(86) International application number: PCT/GB2002/002464
(87) International publication number: WO 2002/098387

(56) References cited:
- EP-A- 0 753 296
- EP-A- 0 945 132
- WO-A-01/41733
- WO-A-99/44580

## Description

This invention relates to formulations adapted to disintegrate in the mouth, to processes to prepare them and to uses thereof.

Non-steroidal anti-inflammatory drugs (NSAIDs) are a widely used class of medicaments. They are a well defined group of compounds and include phenylpropionic acids such as ibuprofen, naproxen, ketoprofen and flurbiprofen. They are primarily used for the treatment of one or more of pain, inflammation and fever, for example rheumatoid arthritis, ankylosing spondylitis, osteoarthritis, postoperative pain, post-partum pain and soft tissue injuries. One example is ibuprofen which is available under prescription in the UK (eg Brufen (RTM)), generally at doses up to 3200 mg per day. Ibuprofen is also available as a non-prescription drug in the UK (eg Nurofen RTM)) primarily for the treatment of symptoms of pain and fever including headache, migraine, rheumatic pain, muscular pain, backache, neuralgia, dysmenorrhoea, dental pain and colds and flu, generally at doses up to 1200 mg per day.

It is desired to provide an NSAID formulation adapted to disintegrate quickly in the mouth. However, a well-known disadvantage of ibuprofen and other NSAIDs is the poor taste and the after-burn sensation in the throat. Thus, expense is incurred, for example in including taste-masking excipients or in processing the formulation in such a way, such as by applying a coating, to reduce the taste of the drug. Due to such considerations, it is difficult to provide an economical process to prepare a tablet formulation containing ibuprofen or other NSAID adapted to disintegrate in the mouth.

US Patent No. 5780046 discloses organoleptically acceptable oral formulations containing S(+)-ibuprofen. The disclosure is based on the discovery that S(+)-ibuprofen does not contain the unpleasant, bitter taste which racemic ibuprofen is known to possess, thus allowing the formulation of a wide variety of dosage forms containing the enantiomer of ibuprofen but without the poor taste. It is preferred that the formulations containing the ibuprofen eutomer also contain an acidic component of sufficient amount to maintain the pH of the formulation below 7.

However, it is desired to provide acceptable orally dispersible formulations with valuable dissolution properties of any of the low-melting NSAIDS without being reduced to using particular forms or derivatives of a drug.

It has been found that if any low-melting NSAID is heated until molten, cooled, formed into solidified melt granules and then combined with an organic acid, advantageous orally dispersible tablets may be formulated.

Accordingly, the present invention provides a pharmaceutical formulation adapted to disintegrate in the mouth comprising a low-melting non-steroidal anti-inflammatory drug and an organic acid, characterised in that the formulation comprises
(a) a granular composition comprising a plurality of solidified melt granules of said non-steroidal anti-inflammatory drug; and
(b) and 5 - 30% w/w of an organic acid.

US 5780046 does not suggest the formation of solidified melt granules of S(+) - ibuprofen.

The term "non-steroidal anti-inflammatory drug" is intended to include the NSAID as a racemic mixture or as a specific enantiomer. It is also intended to include pharmaceutically acceptable salts of the NSAID. Preferred salts are the sodium salts, potassium salts and the lysine salts.

Formulations of the present invention have been found to have a reduced level of the bitter after-taste which is a well-known characteristic of ibuprofen and other NSAIDs. This represents a significant improvement as it removes the requirement to provide a coating over any tablet core or granular composition to mask the taste of NSAID. A formulation according to the invention also reduces the amount of taste-masking excipients which would be required in the absence of a coating. It has also been found that formulations prepared according to the present invention have valuable disintegrating properties in the mouth. Furthermore, the dissolution results show an unexpectedly high level of the NSAID dissolved in the aqueous medium after relatively short periods of time. A further advantage of the present invention lies in the small amount of additional tabletting excipients needed to prepare a dosage form, e.g. to tablet the mixture, thus leading to advantages in processing and cost of the formulations and allowing smaller dosage forms to be produced.

The granular composition comprises a plurality of solidified melt granules of a low-melting NSAID. The melt granules are formed by heating the NSAID until molten. The liquid melt is cooled to a temperature below the melting point of the NSAID and formed into granules. Thus, as used herein, "solidified melt granules" means granules in the form of a solidified melt of NSAID formed by heating said NSAID until molten, optionally combining with excipients, and then forming said NSAID into solidified melt granules.

The formulation is generally expected to comprise 1-90% w/w granular composition, preferably 1-50% w/w, more preferably 1-30% w/w and most preferably 2-20% w/w granular composition.

It has been found that the crystalline form of the NSAID changes on melting and then cooling the NSAID. For example, the crystals become smaller and the surface area of the NSAID in the melt granule is increased compared to that of conventional crystals of the NSAID. In addition, on cooling, the changes to the crystalline structure lead to a more porous granule.

The melt granules are formed by heating the NSAID until molten. This includes heating until fully molten or partially molten. The crystalline structure of the melt granules formed by solidifying fully melted NSAID differs from the crystalline structure where the NSAID is only partially melted. In the case of partial melting, the crystalline structure of the melted NSAID is interrupted by the non-melted NSAID, thus providing that the NSAID comprises more than one crystalline structure. The NSAID is preferably fully melted so that on cooling, a single continuous phase of the NSAID is formed, i.e. the crystalline structure of the NSAID is not interrupted by another crystalline NSAID structure. In preferred compositions according to the present invention, the NSAID is present in a single crystalline state. Also, the melt granules preferably comprise said NSAID as a continuous phase. Accordingly the NSAID continuous phase comprises a single crystalline phase of the NSAID.

The invention allows the formulation of any relatively low-melting NSAID into an acceptably tasting and readily disintegrating formulation in the mouth. A favoured class of compounds are the 2-arylpropionic acids which are generally substantially insoluble and have poor taste properties. It is generally envisaged that the melting point of such compounds will be low enough to allow the melting thereof using standard equipment. It is also important that there is not a deleterious effect on any optional ingredients incorporated in the molten NSAID, for example a disintegrant. Thus, typical melting points of the NSAIDs would be expected to fall within the range 20-300°C. Preferred NSAIDs have lower melting points so that the melting process does not use significant amounts of energy, which thus has an effect on production costs. Preferred melting points are in the range 40-200°C (such as racemic naproxen, melting point 156°C), more preferably 40-150°C, further preferably 40-120°C (such as racemic flurbiprofen, melting point 114°C), most preferably 50-100°C (such as racemic ibuprofen (melting point 75-77°C; S(+)-ibuprofen, melting point 52-54°C and racemic ketoprofen, melting point 96°C). Preferred low-melting NSAIDs are naproxen, ketoprofen, flurbiprofen, ibuprofen and enantiomers thereof. The invention is especially adapted for an ibuprofen and its enantiomers, including mixtures thereof, especially racemic ibuprofen and S(+)-ibuprofen which have low melting points and a very poor after-taste in the mouth and throat. Most advantageous results are obtained with racemic ibuprofen which has a high dosage combined with poor solubility properties.

The proportion of NSAID in the melt granules will depend on the dose desired for a given therapeutic effect. Low dose drugs, such as flurbiprofen and ketoprofen may form as little as 1% by weight if a relatively large dosage form is required. However, particular advantages of the present invention are obtained by allowing a reduction in the number and/or amount of excipients. Accordingly the NSAID may form up to 95% of the melt granules. Accordingly, it is generally envisaged that the melt granules may comprise 1-100% w/w NSAID, more usually 10-100% w/w, preferably 40-100%, more preferably 50-100% w/w and most preferably 60-100 % w/w. A preferred feature of the invention is that low-melting, high dose NSAIDs, such as ibuprofen, can be formulated with minimal excipients into smaller dosage forms. Accordingly, in a most preferred aspect, the melt granules comprise 60-97% w/w of the NSAID, preferably 70-95% and further preferably 75-95% w/w.

In order to accommodate the acid, the NSAID will usually form less than 80% w/w of the formulation, for example 1-80% w/w, suitably 5-60% w/w, preferably 5-50% w/w, more preferably 5-40% w/w, still more preferably 5-30% w/w, most preferably 5-20% w/w and especially 15-25% w/w of the formulation.

Preferred examples of the organic acid include citric, tartaric, malic, fumaric, ascorbic and adipic acids. The formulation comprises 5-30% w/w organic acid, preferably 5-25% w/w, more preferably 10-22% w/w*.* The ratio of NSAID to acid will depend on the proportion of the NSAID in the dosage form. Thus, depending on the dosage of the drug, it can be expected to fall In the range 20:1 to 1:20, conveniently 10:1 to 1:10. For relatively high dose drugs such as ibuprofen and naproxen, the ratio of NSAID to acid may be in the range 2:1 to 1:5, more preferably 1:1 to 1:3, most preferably 1:1 to 9:2. For relatively low dose drugs such as flurbiprofen and ketoprofen, the ratio of NSAID to acid may suitably be 1:1 to 1:20, preferably 1:4 to 1:10. The granular composition may comprise the organic acid or the organic acid may be an extra-granular ingredient in the formulation.

The formulation preferably comprises an extra-granular composition which comprises the ingredients incorporated in the formulation which are not contained in the solidified melt granule. It is preferred that the acid is present in the extra-granular composition. Preferably, the extra-granular composition comprises 5-35% w/w organic acid, more preferably 8-25% w/w and most preferably 10-20% organic acid. The ingredients of the extra-granular composition may be mixed with the melt granules simultaneously or at sequential stages in the process to prepare the formulation. A particular advantage of the present invention is that all the ingredients of the extra-granular composition may be combined with the melt granules at a single stage in the manufacturing process. Also, it is preferred that at this stage, the ingredients in the extra-granular composition are combined sequentially with the melt granules. The formulation comprises a uniform mixture of melt granules and extra-granular composition. The extra-granular composition is suitably distributed evenly throughout the formulation.

Optionally a lubricant may be incorporated in the extra-granular composition for mixing with the melt granules. Conventional lubricants for NSAIDS may be used for example stearic acid, sodium lauryl sulphate, polyethylene glycol, hydrogenated vegetable oil, sodium stearyl fumarate, magnesium stearate or calcium stearate. These may be present in an amount from 0.05-5% by weight, preferably 0.1-2% by weight of the formulation. Anti-adherents such as talc, may further be included in an amount of up to 4% by weight of the dosage form, for example 0.5-2% by weight of the dosage form, preferably as part of the extra-granular component.

The formulation may also comprise a disintegrant. The addition of a disintegrant further improves the rate at which the tablet breaks up when added to a liquid medium. Examples of disintegrants include one or more of starch and modified starch (such as wheat starch, maize starch, potato starch), sodium starch glycolate, low-substituted hydroxypropyl cellulose, alginic acid, cross-linked polyvinylpyrrolidone, magnesium aluminium silicate, bentonite and croscarmellose sodium. Preferred disintegrants are those which swell on the action of water, thus causing the ingredients in the tablet to be pushed apart and out into the aqueous disintegration medium. Preferred examples of disintegrants are croscarmellose sodium and/or sodium starch glycolate, especially croscarmellose sodium. The disintegrant is present at an effective disintegrating amount, for example up to 50% w/w of the formulation (eg 1-50% w/w), more preferably 1-25% w/w, further preferably 2-20% w/w and most preferably 2-15% w/w of the formulation.

The disintegrant is an optional ingredient of the melt granules and/or the extra-granular composition. The disintegrant will suitably form 0.1-25% w/w of the melt granules, preferably 3-15% w/w and most preferably 4-10% w/w of the melt granules. The disintegrant may be present in the extra-granular composition in an amount of 0.1 to 25% w/w, preferably 1-15%, more preferably 2-10% w/w.

The ratio of NSAID to disintegrant will depend on the proportion of the NSAID in the dosage form. Thus, depending on the dosage of the drug, it can be expected to fall in the range 20:1 to 1:20, conveniently 10:1 to 1:10. For relatively high dose drugs, such as ibuprofen and naproxen, the ratio of NSAID to disintegrant may be in the range 20:1 to 2:1, preferably 10:1 to 5:1 parts by weight. For relatively low dose drugs, such as flurbiprofen and ketoprofen, the ratio of NSAID to disintegrant is suitably 1:10 to 10:1, preferably 1:1 to 1:5 parts by weight.

Although not necessary for the production of compositions according to the present invention, if desired, the formulation may further comprise a diluent. The diluent may be water-soluble or water-insoluble. Suitable water-soluble diluent materials include the sugar alcohols (such as xylitol, sorbitol, mannitol, erythritol), sugars (such as sucrose, fructose, lactose, dextrose), cyclodextrin, maltodextrin and salts of organic acids (eg sodium citrate and potassium citrate). Lactose, sodium citrate and potassium citrate are particularly preferred water-soluble diluents. Suitable water-insoluble diluent materials include cellulose derivatives (such as microcrystalline cellulose) starch and derivatives thereof (such as pre-gelatinised starch), dicalcium phosphate, tricalcium phosphate, calcium sulphate, calcium carbonate. Microcrystalline cellulose and dicalcium phosphate are preferred water insoluble diluents. More preferred diluents include microcrystalline cellulose, dicalcium phosphate, xylitol, sorbitol, mannitol, sucrose, lactose and fructose. It is preferred to use water-soluble diluents.

In a formulation adapted to disperse in the mouth, the level of diluent may be quite high, for example up to 90% (such as 0-90% w/w, preferably 0-70% w/w) by weight of the formulation in order to achieve the desired disintegrating properties. Preferably, the diluent forms 1-75% w/w, preferably 10-75% w/w, more preferably 20-75% w/w and most preferably 30-75% w/w of the formulation.

The diluent may be contained in the melt granule or may be part of the extra-granular composition or may be incorporated as desired in both components. If desired, the diluent may preferably be added in an amount up to 90% w/w of the extra-granular composition (ie 0.1-90% w/w), Accordingly, if employed, the diluent may suitably be included in the extra-granular composition in the range 1-90% w/w, preferably 10-90% w/w, more preferably 20-80% w/w and most preferably 30-80% w/w. As discussed hereinabove, the diluent may be present in the melt granules, for example in an amount of 0-30% w/w (such as 0.1-30%) by weight of the melt granules. If present, the diluent conveniently forms 1-20%, more preferably 1-10% w/w of the melt granules. It is preferred to include the diluent in the extra-granular composition.

The formulation may also include a base, preferably an alkali metal salt for example an alkali metal carbonate or bicarbonate, present to an extent of up to 50% by weight (eg in the range 1-50% by weight), preferably up to 40% by weight (eg in the range 1-40% by weight) of the composition (more preferably 2-35% w/w and most preferably 5-20% w/w). The base may react with the organic acid in an effervescent reaction liberating carbon dioxide. Preferably, the alkali metal salt is sodium or potassium. Further preferably, the salt is at least one of sodium bicarbonate, sodium carbonate, potassium bicarbonate and potassium carbonate. Most preferably the base comprises sodium bicarbonate. The ratio of NSAID (especially ibuprofen medicament) to alkali metal salt may be in the range 100:1 to 1:1 parts by weight, preferably 5:1 to 1:1 parts by weight. Preferably, the alkali metal salt is incorporated in any amount up to an equimolar amount with respect to the NSAID (eg ibuprofen). Conveniently, a sub-molar amount of alkali metal salt is incorporated. Thus the alkali metal compound may form up to 100% w/w of the NSAID, preferably 50% w/w, more preferably up to 10% w/w, of the NSAID granules. Preferably the alkali metal salt is on a ratio to said acid of 5:1 to 1:5 parts by weight, more preferably 3:1 to 1:3, most preferably 2:1 to 1:1 parts by weight. Preferably, the extra-granular composition comprises the base.

The formulation may also include a surfactant, in the amount appropriate to the properties of the surfactant, preferably 0.05-10% by weight of the formulation. The surfactant may be included in the melt granules and/or the extra-granular composition. Preferred surfactants are sodium lauryl sulphate, poloxamer, hydrogenated castor oil and derivatives thereof, polyoxyethylene surfactants (including polyoxyethylene oils, fatty acid esters, including stearates) and sorbitan esters. A surfactant may be used to an extent of 0.05-5% w/w, preferably 0.1-3% w/w, more preferably 0.2-2% w/w of either or both the melt granules and the extra-granular composition.

The formulation may also comprise flow aids such as silicon dioxide and talc. Most preferably the silicon dioxide is colloidal silicon dioxide (especially having a mean particle size less than 50nm such as 5-40nm), most preferably anhydrous colloidal silicon dioxide. The silicon dioxide may be incorporated in the composition to an extent of 0.05-5.0% w/w, preferably 0.1-3% by weight, more preferably 0.2-1% w/w of the composition. The melt granules and/or the extra-granular composition may comprise the flow aid.

Other conventional tabletting excipients such as gelling agents and/or binders known to the person skilled in the art may be incorporated in the formulation, for example a compressed tablet composition, as desired (although it will be appreciated that a prime advantage of the present invention is that the number of excipients necessary to achieve a quickly disintegrating formulation with good dissolution characteristics is minimal). Examples include gelling agents, such as gelatin, carageenan, tragacanth, guar gum; and binders such as PVP, Pregelled maize starch, HPMC, dextrin, liquid glucose. Gelling agents may suitably used in an amount of 0.1-10% of the formulation, preferably 1-5% of the formulation. Binders may suitably used in an amount of 0.1-20% of the formulation, preferably 1-10% w/w of the formulation. It may also be desired to add appropriate amounts of flavours and/or sweeteners to enhance the taste. These ingredients may form 0-10% w/w of the formulation, preferably 0-5% w/w. Conveniently, the extra-granular composition comprises the flavours and/or sweeteners in an amount of 0-10% w/w, more preferably 0-5% w/w. Although not necessary for the carrying out of the present invention, if desired the formulation may comprise additional excipients conveniently used in formulations adapted to disintegrate in the mouth. If desired, the extra-granular material may also comprise a masticatory material or other release-modifying excipients which are adapted to release the ibuprofen in a desired way according to its desired use.

The NSAID (preferably ibuprofen) melt granules optionally containing a disintegrant are preferably combined with an extra-granular composition comprising an acid, an alkali metal salt, a diluent and optionally a surfactant and or other tabletting excipients. Accordingly, in one preferred embodiment, the melt granules may comprise greater than 90% w/w of the NSAID (ie 90-100% w/w). Preferred melt granules comprise an NSAID (preferably ibuprofen), a disintegrant and optionally a surfactant and/or a diluent. In a further preferred embodiment, the melt granules comprise greater than 90% w/w (ie 90-100% w/w) of the combination of NSAID and disintegrant. Further preferably, the melt granules consist essentially of (i.e. 98-100% w/w) of the combination of NSAID and a disintegrant. Further preferred melt granules consist essentially of an NSAID (preferably ibuprofen), a disintegrant, and a diluent.

In a preferred formulation according to the present invention the melt granules comprise:
(a) 85-95% w/w non-steroidal anti-inflammatory drug, and
(b) 5-15% w/w disintegrant.

In an especially preferred formulation according to the present invention the melt granules consist essentially of:
(a) 85-95% w/w non-steroidal anti-inflammatory drug, and
(b) 5-15% w/w disintegrant.

An advantageous formulation (especially a tablet formulation) according to the present invention comprises an extra-granular composition comprising an acid, a base, a diluent including a sugar and/or sugar alcohol (or mixtures thereof), and a lubricant and/or a surfactant and/or flavours and/or sweeteners. A particularly preferred composition comprises 10-90% w/w diluent, 5-30% w/w acid, 5-30% w/w base and 0-5% w/w lubricant. This combination suitably forms an intimate admixture with said melt granules prior to filling into a unit dose (eg a sachet or capsule) or compression into a tablet.

The extra-granular composition preferably comprises at least one of silicon dioxide, stearic acid or a salt thereof, a surfactant and a diluent. More preferably, the extra-granular composition consists essentially of an acid, sodium bicarbonate, a lubricant and a diluent including at least one of a sugar and sugar alcohol.

A preferred formulation according to the invention comprises:
(I) a granular composition comprising:
   (a) 85-95% w/w non-steroidal anti-inflammatory drug, and
   (b) 5-15% w/w disintegrant
      and
(II) an extra-granular composition comprising:
   (c) 10-90% w/w diluent;
   (d) 5-30% w/w acid;
   (e) 5-30% w/w base, and
   (f) 0-5% w/w surfactant.

The formulation most preferably comprises a combination of melt granules of ibuprofen with an extra-granular composition comprising an acid selected from citric acid, malic acid and/or tartaric acid; a lubricant comprising stearic acid or a salt thereof; an alkali metal salt; and a diluent comprising at least one of a sugar and a sugar alcohol. This combination may take the form of a uniform or homogeneous blend capable of being mixed with other ingredients as desired and formed into a unit dose. The unit dose is arranged to disperse in the mouth. The preferred embodiment is a dosage form in the form of a compressed tablet. The tablet may be swallowed or retained in the mouth (for example chewed or allowed to disintegrate in the mouth).

The dosage form may be sucked or it may be chewed up. The therapeutic effect is achieved more quickly if the dosage form is crunched up and swallowed substantially immediately. A typical dosage form could be chewed and swallowed within two minutes, preferably less than one minute.

The present invention preferably provides a chewable tablet adapted to disintegrate in the mouth and comprising:
(a) a granular composition comprising a plurality of solidified melt granules of a low-melting non-steroidal anti-inflammatory drug; and
(b) an extra-granular composition comprising an organic acid.

In other words, there is provided a chewable tablet that comprises a formulation according to the present invention.

A preferred formulation according to the present invention comprises:
(a) 5-30%, preferably 5-25 % w/w ibuprofen;
(b) 5-30% w/w acidic component;
(c) 0.1-5% w/w croscarmellose sodium;
(d) 5-20% w/w sodium bicarbonate;
(e) 50-70% w/w diluent.

The formulations of the invention such as compressed tablet compositions may, if desired, include other compatible pharmacologically active ingredients and/or enhancing agents. Thus, for example, the dosage form may include any other ingredient commonly used in a composition useful to treat pain, inflammation and/or fever, for example caffeine or another xanthine derivative, another analgesic, for example codeine, a skeletal muscle relaxant: an antihistamine (e.g. acrivastine, astemizole, azatadine, azelastine, bromodiphenhydramine, brompheniramine, carbinoxamine, cetirizine, chlorpheniramine, cyproheptadine, dexbromopheniramine, dexchloropheniramine, diphenhydramine, ebastine, ketotifen, lodoxamide, loratidine, levocabastine, mequitazine, oxatomide, phenindamine, phenyltoloxamine, pyrilamine, setastine, tazifylline, temelastine, terfenidine, tripelennamine or triprolidine (preferably non-sedating antihistamines are employed)); a decongestant (eg pseudoephedrine, phenylpropanolamine and phenylephrine); a cough suppressant (eg caramiphen, codeine or dextromethorpan); and/or an expectorant (eg guaifenesin, potassium citrate, potassium guaiacolsuphonate, potassium sulphate and terpin hydrate).

Such extra active ingredients and/or enhancing agents may be incorporated in the melt granules or in the extra-granular composition in appropriate dosage amounts for the desired therapeutic effect. Reference may be made to MIMS and the Physicians Desk Reference for guidelines as to a suitable dosage. It is generally expected that such other active ingredients will form 0-50% w/w of the formulation, for example 5-25% w/w.

Ibuprofen and its derivatives are primarily anti-inflammatory, analgesic and antipyretic agents but have also been proposed for other therapeutic uses, including the treatment of periodontal bone loss, pruritus and Alzheimers disease. The dosage forms of the present invention are therefore indicated for use in the treatment of all therapeutic uses for which ibuprofen is effective, including rheumatoid arthritis, osteoarthritis, ankylosing spondylitis, seronegative arthropathies, periarticular disorders and soft tissue injuries. They may also be used in the treatment of postoperative pain, postpartum pain, dental pain, dysmenorrhoea, headache, migraine, rheumatic pain, muscular pain, backache, neuralgia and/or musculoskeletal pain or the pain or discomfort associated with the following: respiratory infections, colds or influenza, gout or morning stiffness. Especially preferred uses include coughs, colds, influenza, migraine, headache, rheumatic pain, muscular pain and neuralgia.

Accordingly, in another aspect of the present invention there is provided a composition according to the present invention for use in the treatment of pain and/or inflammation and/or fever. Furthermore, the invention also provides a method of treating pain and/or inflammation and/or fever comprising the administration of a composition according to the present invention to a mammal in need thereof. The formulations of the invention can be used in the manufacture of medicaments for use in these methods.

Unit dosages for effective therapy are known to those skilled in the art for each NSAID. For example, they may comprise the NSAID to an extent of 5mg, 10mg, 12.5mg, 25mg, 50mg, 100mg, 150mg, 200mg, 250mg, 300mg, 350mg, 400mg, 500mg, 600mg and 800mg. Where derivatives are employed, normally the precise unit dosages are chosen to give the equivalent NSAID doses given above. For the treatments described herein the maximum daily dose of ibuprofen is generally 3200 mg. A single unit daily dose may be 100 mg. Preferred unit doses are in the range 100-400 mg, more preferably 100-300 mg and especially 200 mg ibuprofen. The maximum daily dose of flurbiprofen is generally 300 mg. A single unit dose may be 12.5 mg. Preferred unit doses are in the range 12.5-150 mg, more preferably 25-100 mg and especially 50 mg flurbiprofen. The maximum daily dose of naproxen is generally 1500 mg. A single unit daily dose may be 125 mg. Preferred unit doses are in the range 220-750 mg, more preferably 220-500 mg and especially 220-250 mg naproxen. The maximum daily dose of ketoprofen is generally 200 mg. A single unit dose may be 25 mg. Preferred unit doses are in the range 25-100 mg, more preferably 25-75 mg and especially 50 mg ketoprofen.

In a further aspect, the present invention provides a process to prepare a formulation according to the invention characterised by the following steps :
(a) melting said non-steroidal anti-inflammatory drug optionally with one or more excipients;
(b) forming said mixture into solidified melt granules;
(c) combining said solidified melt granules with an organic acid to form a uniform mixture; and optionally
(d) forming into a unit dose.

In step (a) of a process according to the present invention, the drug is melted. At room temperature, the NSAIDs used in accordance with the present invention are solid. Thus, it is necessary to heat the NSAID until it is in the molten state. Under pressurised conditions, the drug may be melted at a temperature below its normal melting point. Melting may be carried out according to known methods, including for example, heating in a vessel to a temperature above the melting point of the NSAID or by extrusion in a heated extruder. The maximum temperature is determined by the stability of the molten drug and ingredients combined therewith. The drug may be heated to any convenient temperature. Generally, the higher the temperature, the more quickly the drug will melt although this must be balanced by the energy input required to heat the drug. For highest efficiency, it is generally envisaged that the NSAID will be heated to not more than 50°C, preferably 1-25°C and more preferably 5-20°C, above its melting point to keep energy costs to a minimum. A preferred heating range is 30-180°C, more preferably 35-140°C and more preferably 40-120°C.

If the NSAID is extruded, generally the work on the NSAID by the screw configuration in the extruder will also contribute to melting the NSAID, thereby reducing its external applied temperature requirement. Accordingly, the extruder barrel may be heated to a temperature less than the melting point of the NSAID. For example, the normal melting point of racemic ibuprofen is 75-77°C, however under conditions of force/pressure (such as may be encountered in an extruder or similar processing device), the external applied heat necessary to melt the ibuprofen may be reduced significantly through the mechanical heat generated by the intense mixing action within the extruder. It is generally envisaged that the extruder will be heated to a temperature not less than 25°C below the melting point of the NSAID, preferably in the range from 20°C below the melting point of the drug to 50°C above the melting point of the drug, more preferably from 15°C below the melting point of the drug to 25°C above its melting point and most preferably to a temperature in the range of 10°C on each side of the melting point of the drug. Some extruders allow different zones to be heated to different temperatures in the extruder. These temperatures can be chosen as desired to ensure that the NSAID is melted, preferably fully melted, in stage (a). Preferably, the drug is heated to a temperature in the range 80-130°C, more preferably 100-120°C. When the NSAID is ibuprofen it may conveniently be heated in the range 50-130°C, more preferably 60-100°C. When heated by conventional heating means such as a water or steam bath, it is preferably heated in the range 75-90°C, more preferably 75-85°C. The ibuprofen may also be heated and subjected to conditions of force, such as by heat-extruding the ibuprofen, for example in a twin-screw extruder. The temperature of the ibuprofen in the extruder barrel is preferably in the range 66-96°C, preferably 70-82°C. Any optional excipients may be dispersed throughout the melt by conventional blending and/or stirring techniques to form a molten mixture.

Preferably a disintegrant may be combined with the drug in step (a). Alternatively or additionally, a surfactant may be combined with the drug in step (a). The disintegrant and/or surfactant may be added before or after the drug is melted.

Conveniently, heating the NSAID and blending of optional excipients (eg by stirring, agitating, kneading or extruding the NSAID) occurs at the same time. The above-mentioned process may be carried out in a number of ways. The manner in which optional excipients are combined with the drug will depend on conditions, including the drug used and the dosage thereof, the temperature to which the drug is heated, the amount of any other excipients used, the quantity of ingredients being processed and will be within the knowledge of the person skilled in the art. In one method, the NSAID is heated in a suitable vessel until molten. The optional components may then be added to the molten mass and thoroughly combined therewith to form a homogeneous mixture. These optional extra excipients may also be blended into the molten NSAID simultaneously or in sequential steps.

In a further process, the non-steroidal anti-inflammatory drug may be combined in the solid state with the optional excipients, eg a disintegrant, and then heated together until said non-steroidal anti-inflammatory drug is molten. It may also be desired to combine the NSAID with at least one additional excipient in the solid state, then heat until said NSAID is molten and then add any further desired excipients.

In another method, the NSAID and any optional ingredients to be incorporated in the melt granule are fed into an extruder type system (preferably having first been combined by blending together). The materials are heated and mixed by kneading by the screw(s) within the extruder until the NSAID is molten and a uniform mixture of the components is produced. Preferably, the drug is melt extruded. Further preferably, the NSAID is extruded in a twin screw extruder.

In a preferred process, the disintegrant is combined with the melted NSAID, either prior to melting or after the melting process. The disintegrant is most commonly insoluble in the ibuprofen melt and a dispersion of the solid disintegrating agent within the liquid melt is produced. The dispersion is mixed so that the disintegrating agent is uniformly or homogeneously combined with the melted NSAID. A uniform mixture is thus produced.

In step (b), the molten mixture is formed into solidified melt granules. The melt is allowed to solidify in any manner convenient. In some cases, the melt granules may be formed whilst the drug is in the molten state (i.e. fully molten or partially molten) and then allowed to cool to form the solidified melt granules. This may be carried out, for example in a melt-extrusion process, for example the extrudate may be chopped into pellets as it passes out of the extruder and the pellets cooled in an appropriate manner, such as a cooling belt. In a further method, after heating or heat-extrusion the molten NSAID may be cooled by feeding either to a spray tower dryer or to a spray granulator in which the molten mass is sprayed into the path of a stream of cold air to form droplets and the dried solid mass collected.

Generally, it is expected that the molten mixture will be cooled to a temperature below the melting point of the drug before being formed into granules. This includes both rapid cooling and slow cooling. Preferably the molten drug is cooled rapidly. For example, the molten NSAID may be allowed to cool at room temperature or in a cooled vessel (eg water-cooled). The molten NSAID may also be poured onto cooling trays which may be static or continuously moving. Static trays may be placed in cooling cabinets. The cooled melt forms a solid and may be scraped off the belt or collected as it falls off one end of a continuously moving belt. In order to maintain a uniform mixture of the drug with any excipients, it may be necessary to agitate or stir the mixture during cooling. Cooling may also occur in a stream of cooled air. The molten drug may also be cooled by passing the molten mixture onto a moving cooling belt, preferably a continuously rotating cooling belt. Preferably, the belt is cooled by water. The water may be applied to the underside of the belt along its length or partially along its length as desired and according to the length of the belt, the quantity of molten drug mixture and the speed of the belt. It is especially preferred to cool the molten drug mixture at least initially by cooling means, for example until it has started to solidify. Advantageously, the belt is water-cooled along substantially the whole of its length and it is of minimum length required (e.g. 3-7m) to allow it to cool to the solid state.

The granules produced on cooling the molten drug are preferably of a suitable size for tabletting, preferably in a standard large scale tabletting machine. The melt granules in the granular composition preferably have a mean particle size in the range 10-2000µm, more preferably 50-1000µm and most preferably 100-400µm. Valuable results are achieved when the bulk density of the melt granules is in the range 0.1-1gml⁻¹ more preferably 0.3-0.6gml⁻¹. Further preferred properties are obtained when the tapped density is in the range 0.3-0.7gml⁻¹ (more preferably 0.4-0.6 gml⁻¹). Further, preferably the melt granules have a porosity of 0.5-2.0 g/ml.

The solidified melt may be formed into granules by a plurality of methods. For example, it may be pulverised or comminuted into granules. It may be milled and/or sieved. If it is cooled on a moving belt, the cooled melt may be delivered to a comminuting device such as a scraper bar and/or mill. It may also be passed through a spray device such as a spray tower or spray granulator in which the molten material is sprayed from an orifice into a stream of cooled air, allowed to congeal/solidify and then collected. If the molten NSAID is extruded, the extrudate may be cooled and then broken into conveniently sized pieces, followed by milling and or sieving. Alternatively, the extrudate may be extruded through holes and chopped into suitably sized granules for tabletting.

Preferably in stage (b) the molten drug mixture is formed into a ribbon or ribbons and is cooled on a water-cooled belt. The solidified ribbons may be milled into granules. The granular composition may be sieved to ensure that the melt granules are of the appropriate size for efficient tabletting.

In step (c), the solidified melt granules are combined with an organic acid. This may be achieved by conventional mixing and blending techniques. Examples of apparatus that may be used to facilitate this process are Ribbon Blender, IBC Blender, V-Blender & Plough Blenders.

The melt granules are combined thoroughly so as to form a uniform mixture of ingredients. The acid should be distributed evenly through the formulation to maximise the taste properties.

The combined melt granules and extra granular composition may be formed into a unit dose. Examples include filling of the loose powder mixture into a sachet or a capsule or compressing it into a tablet. Chewable tablets are the preferred unit dosage form according to the invention. It has been found that the taste of the NSAID has been substantially masked which allows the dosage form to be maintained in the oral cavity for a period of time whilst the formulation disintegrates.

In a preferred feature of step (c), an extra-granular composition comprises the organic acid and at least one of a diluent and a lubricant.

In a preferred process according to the present invention, said NSAID comprises Ibuprofen.

In a further aspect of the invention, there is provided a process to prepare a granular composition comprising a plurality of melt granules of a low-melting non-steroidal anti-inflammatory agent characterised by :
(a) melting said drug until fully molten;
(b) cooling to the solid state; and
(c) forming solidified melt granules.

The invention is illustrated by the following non-limiting Examples. In the Examples, the racemic ibuprofen is available from BASF Pharma, Bishop, TX, USA; colloidal silicon dioxide (also known as colloidal silica) is available from Degussa, Frankfurt, DE under the trade name Aerosil 200; Croscarmellose sodium is available from the FMC Corporation, Brussels, BE under the tradename Ac-Di-Sol; and sodium starch glycollate is available from Edward Mendell, Reigate, GB under the tradename Explotab.

### Dissolution Measurement

The dissolution was measured using the dissolution method described in the US Pharmacopoeia Vol. 23, page 1791, Apparatus 2 using paddles at 50 rpm and a phosphate buffer (selected at pH 7.2 and/or pH 6.0 and/or pH 5.8).

### Friability Measurement

This test for the robustness of the tablet is a standard friability test, namely the rotation of 20 tablets for 4 minutes at 25rpm in a friabulator (TAR 20 manufactured by ERWEKA). The number of capped or broken tablets may be measured.

### Crushing Strength (N)

The crushing strength is a measure of the hardness of a tablet. It may be measured by recording the diametrical crushing strength when the tablet is broken between the motorised jaws of a Schleuniger crushing strength tester.

### Disintegration Time (Minutes)

The disintegration time may be measured using the disintegration method described in the European Pharmacopoeia 1986, Ref V.5.1.1 (updated 1995) using tap water (pH approximately 7) as the liquid. The method measures the time (seconds) by which six tablets prepared with each Example formulation disintegrates.

### Example 1(a): Preparation of Granular Composition

| | | Ex 1 (% w/w) |
|---|---|---|
| Granular Composition: | | |
| | Ibuprofen | 10.0 |
| | Croscarmellose Sodium | 0.8 |

| Extra-granular material: | | |
|---|---|---|
| | Xylitol | 52.0 |
| | Malic acid | 20.0 |
| | Sodium bicarbonate | 15.0 |
| | Magnesium stearate | 1.0 |
| | Flavourings | 1.0 |

In the illustrative process, the ibuprofen was first melted by heating to approximately 75°C in a stainless steel vessel on a steam bath until molten. The disintegrating agent (croscarmellose sodium) was then added to the molten mass, and mixed for 5-10 minutes until uniformly dispersed. The molten mixture was allowed to cool and crystallise. The solidified melt was removed from the vessel, ground and milled by passing through a cone mill having a screen with a round hole size of 1 mm. The milled mass was collected.

### Example 1(b): Preparation of Tablets

The extra-granular basing ingredients, namely a filler, an organic acid, a lubricant, a base and flavourings and sweeteners, blended with the granular composition for approximately 15 minutes in a blender. The blended material was fed to a tabletting machine and compressed into tablets containing 200mg ibuprofen.

In the same way, tablets containing 50mg, 100mg, 150mg, 300mg and 400mg ibuprofen or S(+)-ibuprofen can be prepared. Optionally, an inert diluent such as a conventional sugar and/or cellulose material may also be incorporated, as the amount of S(+)-ibuprofen could be reduced compared to the amount of racemic ibuprofen normally incorporated into solid dosage forms.

### Examples 2-4

| | | **Ex 2 (% w/w)** | **Ex 3 (% w/w)** | **Ex 4 (% w/w)** |
|---|---|---|---|---|
| Granular Composition: | | | | |
| | Ibuprofen | 10.0 | 10.0 | 10.0 |
| | Croscarmellose Sodium | 0.8 | 0.8 | 0.8 |

| Extra-granular material: | | | | |
|---|---|---|---|---|
| | Xylitol | 57.0 | 51.8 | 56.5 |
| | Malic acid | 15.0 | 20.0 | 17.5 |
| | Sodium bicarbonate | 15.0 | 15.0 | 12.5 |
| | Magnesium stearate | 1.0 | 1.0 | 1.0 |
| | Flavourings | 1.0 | 1.2 | 1.5 |
| | Sweeteners | 0.3 | 0.3 | - |

### Examples 5-9

| | | **Ex 5 (%w/w)** | **Ex 6 (%w/w)** | **Ex 7 (%w/w)** | **Ex 8 (%w/w)** |
|---|---|---|---|---|---|
| Granular Composition: | | | | | |
| | Ibuprofen | 10.0 | 10.0 | 9.8 | 9.8 |
| | Croscarmellose Sodium | 0.8 | 0.8 | 0.8 | 0.8 |

| Extra-granular material: | | | | | |
|---|---|---|---|---|---|
| | Xylitol | 59.2 | 61.5 | 60.3 | 60.3 |
| | Tartaric acid | - | - | 14.7 | - |
| | Citric acid | - | - | - | 14.7 |
| | Malic acid | 16.3 | 15.0 | - | - |
| | Sodium bicarbonate | 11.3 | 10.0 | 9.8 | 9.8 |
| | Magnesium stearate | 1.0 | 1.0 | 1.0 | 1.0 |
| | Flavourings | 1.5 | 1.5 | 1.4 | 1.4 |
| | Sweeteners | 0.3 | 0.3 | 0.2 | 0.2 |

### Examples 9-10

| | | **Ex 9 (% w/w)** | **Ex 10 (% w/w)** |
|---|---|---|---|
| Granular composition: | | | |
| | Ibuprofen | 10.0 | 10.0 |
| | Croscarmellose Sodium | 0.8 | 0.8 |

| Extra-granular material | | | |
|---|---|---|---|
| | Xylitol | 61.5 | 61.5 |
| | Tartaric acid | 15.0 | 15.0 |
| | Sodium bicarbonate | 10.0 | 10.0 |
| | Magnesium stearate | 1.0 | 1.0 |
| | Flavourings | 1.4 | 1.4 |
| | Sweeteners | 0.3 | 0.3 |

### Examples 11-13

| | | **Ex 11 (% w/w)** | **Ex 12 (% w/w)** | **Ex 13 (% w/w)** |
|---|---|---|---|---|
| Granular composition: | | | | |
| | Ibuprofen | 10.0 | 10.0 | 10.0 |
| | Croscarmellose Sodium | 0.8 | 0.8 | 0.8 |

| Extra-granular material: | | | | |
|---|---|---|---|---|
| | Xylitol | 61.0 | 59.0 | 61.0 |
| | Lactose | - | - | 0.5 |
| | Malic acid | 15.0 | 15.0 | 15.0 |
| | Sodium bicarbonate | 10.0 | 10.0 | 10.0 |
| | Microcrystalline cellulose | 0.5 | 2.5 | - |
| | Magnesium stearate | 1.0 | 1.0 | 1.0 |
| | Flavourings | 1.4 | 1.4 | 1.4 |
| | Sweeteners | 0.3 | 0.3 | 0.3 |

### Examples 14-16

| | | **Ex 14 (% w/w)** | **Ex 15 (% w/w)** | **Ex 16 (% w/w)** |
|---|---|---|---|---|
| Granular composition: | | | | |
| | Ibuprofen | 10.0 | 10.0 | 10.0 |
| | Croscarmellose sodium | 0.8 | 0.8 | 0.8 |
| | Microcrystalline cellulose | - | - | 1.0 |
| | Lactose | - | 1.3 | - |

| Extra-granular material: | | | | |
|---|---|---|---|---|
| | Xylitol | 60.5 | 8.9 | 61.2 |
| | Lactose | 1.0 | 1.3 | - |
| | Malic acid | 15.0 | 15.0 | 15.0 |
| | Sodium bicarbonate | 10.0 | 10.0 | 10.0 |
| | Magnesium stearate | 1.0 | 1.0 | 1.0 |
| | Flavourings | 1.4 | 1.4 | 1.4 |
| | Sweeteners | 0.3 | 0.3 | 0.3 |

The dissolution results for Example 14 at pH 7.2 is given below:-

| **Time (min)** | **Ex 14** |
|---|---|
| 0 | 0.0% |
| 10 | 86.9% |
| 20 | 91.3% |
| 30 | 91.6% |
| 45 | 91.7% |
| 60 | 90.3% |

### Examples 17-19

| | | **Ex 17 (% w/w)** | **Ex 18 (% w/w)** | **Ex 19 (% w/w)** |
|---|---|---|---|---|
| Granular composition: | | | | |
| | Ibuprofen | 10.0 | 10.0 | 10.0 |
| | Croscarmellose sodium | 0.8 | 0.8 | 0.8 |
| | Dicalcium phosphate | 2.5 | - | 0.5 |

| Extra-granular material: | | | | |
|---|---|---|---|---|
| | Xylitol | 59.0 | 61.0 | 60.5 |
| | Malic acid | 15.0 | 15.0 | 15.0 |
| | Sodium bicarbonate | 10.0 | 10.0 | 10.0 |
| | Dicalcium phosphate | - | 0.5 | 0.5 |
| | Magnesium stearate | 1.0 | 1.0 | 1.0 |
| | Flavourings | 1.4 | 1.4 | 1.4 |
| | Sweeteners | 0.3 | 0.3 | 0.3 |

### Examples 20-23

| | | **Ex 20 (% w/w)** | **Ex 21 (%w/w)** | **Ex 22 (%w/w)** | **Ex 23 (% w/w)** |
|---|---|---|---|---|---|
| Granular Composition: | | | | | |
| | Ibuprofen | 10.0 | 10.0 | 10.0 | 10.0 |
| | Polyvinyl pyrrolidone | - | 0.8 | - | - |
| | Croscarmellose sodium | - | - | 0.5 | 1.5 |
| | Sodium Starch glycollate | 0.8 | - | - | - |

| Extra-granular material: | | | | | |
|---|---|---|---|---|---|
| | Xylitol | 61.5 | 61.5 | 61.8 | 60.8 |
| | Malic acid | 15.0 | 15.0 | 15.0 | 15.0 |
| | Sodium bicarbonate | 10.0 | 10.0 | 10.0 | 10.0 |
| | Magnesium stearate | 1.0 | 1.0 | 1.0 | 1.0 |
| | Flavourings | 1.4 | 1.4 | 1.4 | 1.4 |
| | Sweeteners | 0.3 | 0.3 | 0.3 | 0.3 |

### Examples 24-26

| | | **Ex 24 (% w/w)** | **Ex 25 (%w/w)** | **Ex 26 (%w/w)** |
|---|---|---|---|---|
| Granular composition: | | | | |
| | Ibuprofen | - | 10.0 | 13.3 |
| | Flurbiprofen | 10.0 | - | - |
| | Croscarmellose sodium | 0.8 | 0.8 | 1.1 |

| Extra-granular material: | | | | |
|---|---|---|---|---|
| | Xylitol | 61.5 | 76.5 | - |
| | Sorbitol | - | - | 68.8 |
| | Malic acid | 15.0 | - | 13.3 |
| | Citric acid | - | 10.0 | - |
| | Sodium bicarbonate | 10.0 | - | - |
| | Magnesium stearate | 1.0 | 1.0 | 1.3 |
| | Flavourings | 1.4 | 1.4 | 1.9 |
| | Sweeteners | 0.3 | 0.3 | 0.3 |

### Examples 27-29

| | | **Ex 27 (% w/w)** | **Ex 28 (% w/w)** | **Ex 29 (% w/w)** |
|---|---|---|---|---|
| Granular composition: | | | | |
| | Ibuprofen | 10.0 | 10.0 | 10.0 |
| | Croscarmellose cellulose | 0.8 | 0.8 | 0.8 |

| Extra-granular material: | | | | |
|---|---|---|---|---|
| | Mannitol | - | - | 61.5 |
| | Fructose | - | 61.5 | - |
| | Sucrose | 61.5 | - | - |
| | Citric acid | 15.0 | 15.0 | 15.0 |
| | Magnesium stearate | 1.0 | 1.0 | 1.0 |
| | Flavourings | 1.4 | 1.4 | 1.4 |
| | Sweeteners | 0.3 | 0.3 | 0.3 |
| | | | | |

### Examples 30-32

| | | **Ex 30 (% w/w)** | **Ex 31 (% w/w)** | **Ex 32 (% w/w)** |
|---|---|---|---|---|
| Granular composition: | | | | |
| | Ibuprofen | 10.0 | 13.2 | 13.2 |
| | Croscarmellose cellulose | 0.8 | 1.1 | 1.1 |

| Extra-granular material: | | | | |
|---|---|---|---|---|
| | Lactose | 61.5 | - | - |
| | Sucrose | - | 68.9 | - |
| | Fructose | - | - | 68.9 |
| | Citric acid | 15.0 | 13.2 | 13.2 |
| | Magnesium stearate | 1.0 | 1.3 | 1.3 |
| | Flavourings | 1.4 | 2.0 | 2.0 |
| | Sweeteners | 0.3 | 0.3 | 0.3 |

### Examples 33-36

| | | **Ex 33 (%w/w)** | **Ex 34 (% w/w)** | **Ex 35 (% w/w)** | **Ex 36 (%w/w)** |
|---|---|---|---|---|---|
| Granular Composition: | | | | | |
| | Ibuprofen | 13.2 | 13.2, | 10.0 | 10.0 |
| | Croscarmellose sodium | 1.1 | 1.1 | 0.8 | 0.8 |
| | Colloidal silicon dioxide | - | - | 0.1 | - |

| Extra-granular material: | | | | | |
|---|---|---|---|---|---|
| | Mannitol | 68.9 | - | - | - |
| | Xylitol | - | - | 61.4 | 61.4 |
| | Lactose | - | 68.9. | - | - |
| | Tartaric acid | - | - | 15.0 | 15.0 |
| | Citric acid | 13.2 | 13.2 | - | - |
| | Sodium bicarbonate | - | - | 10.0 | 10.0 |
| | Magnesium stearate | 1.3 | 1.3 | 1.0 | 1.0 |
| | Flavourings | 2.0 | 2.0 | 1.4 | 1.4 |
| | Sweeteners | 0.3 | 0.3 | 0.3 | 0.3 |
| | Colloidal silicon dioxide | - | - | - | 0.1 |

### Examples 37-40

| | | **Ex 37 (%w/w)** | **Ex 38 (%w/w)** | **Ex 39 (%w/w)** | **Ex 40 (% w/w)** |
|---|---|---|---|---|---|
| Granular Composition: | | | | | |
| | Ibuprofen | 10.0 | 10.0 | 10.0 | 10.0 |
| | Croscarmellose sodium | 0.8 | 0.8 | 0.8 | 0.8 |

| Extra-granular material: | | | | | |
|---|---|---|---|---|---|
| | Xylitol | 66.5 | 46.5 | 71.5 | 51.5 |
| | Tartaric acid | 15.0 | 15.0 | 5.0 | 25.0 |
| | Sodium bicarbonate | 5.0 | 25.0 | 10.0 | 10.0 |
| | Magnesium stearate | 1.0 | 1.0 | 1.0 | 1.0 |
| | Flavourings | 1.4 | 1.4 | 1.4 | 1.4 |
| | Sweeteners | 0.3 | 0.3 | 0.3 | 0.3 |

### Examples 41-43

| | | **Ex 41 (% w/w)** | **Ex 42 (% w/w)** | **Ex 43 (% w/w)** |
|---|---|---|---|---|
| Granular composition: | | | | |
| | Flurbiprofen | 2.5 | - | - |
| | Ibuprofen | - | 10.0 | 10.0 |
| | Croscarmellose sodium | 0.8 | 0.8 | 0.8 |

| Extra-granular material: | | | | |
|---|---|---|---|---|
| | Xylitol | 69.0 | 62.9 | - |
| | Sucrose | - | - | 62.9 |
| | Citric acid | 15.0 | 15.0 | 15.0 |
| | Sodium bicarbonate | 10.0 | 10.0 | 10.0 |
| | Magnesium stearate | 1.0 | 1.0 | 1.0 |
| | Flavourings | 1.4 | 0 | 0 |
| | Sweeteners | 0.3 | 0.3 | 0.3 |

### Examples 44-46

| | | **Ex 44 (% w/w)** | **Ex 45 (% w/w)** | **Ex 46 (% w/w)** |
|---|---|---|---|---|
| Granular composition: | | | | |
| | Ibuprofen | 10.0 | 10.0 | 10.0 |
| | Sodium starch glycolate | 1.5 | 2.5 | - |
| | Crospovidone | - | - | 1.5 |

| Extra-granular material: | | | | |
|---|---|---|---|---|
| | Sucrose | 60.8 | 59.8 | 60.8 |
| | Citric acid | 15.0 | 15.0 | 15.0 |
| | Sodium bicarbonate | 10.0 | 10.0 | 10.0 |
| | Magnesium stearate | 1.0 | 1.0 | 1.0 |
| | Flavourings | 1.4 | 1.4 | 1.4 |
| | Sweeteners | 0.3 | 0.3 | 0.3 |

### Examples 47-49

| | | **Ex 47 (% w/w)** | **Ex 48 (% w/w)** | **Ex 49 (% w/w)** |
|---|---|---|---|---|
| Granular composition: | | | | |
| | Ibuprofen | 10.0 | 10.0 | 28.6 |
| | Croscarmellose sodium | - | - | 5.2 |
| | Crospovidone | 2.5 | - | - |
| | Maize starch | - | 2.5 | - |

| Extra-granular material: | | | | |
|---|---|---|---|---|
| | Sucrose | 59.8 | 59.0 | - |
| | Xylitol | - | - | 35.4 |
| | Citric acid | 15.0 | 15.0 | 14.3 |
| | Sodium bicarbonate | 10.0 | 10.0 | 10.7 |
| | Magnesium stearate | 1.0 | 1.0 | 1.0 |
| | Flavourings | 1.4 | 1.4 | 4.1 |
| | Sweeteners | 0.3 | 0.3 | 0.7 |
| | Croscarmellose sodium | - | 0.8 | - |

### Examples 50-52

| | | **Ex 50 (%w/w)** | **Ex 51 (%w/w)** | **Ex 52 (%w/w)** |
|---|---|---|---|---|
| Granular composition: | | | | |
| | Ibuprofen | 28.6 | 28.6 | 28.6 |
| | Croscarmellose sodium | 5.2 | 4.3 | 5.2 |

| Extra-granular material: | | | | |
|---|---|---|---|---|
| | Xylitol | - | 25.6 | 42.6 |
| | Sorbitol | 35.4 | - | - |
| | Citric acid | 14.3 | 21.4 | 10.7 |
| | Sodium bicarbonate | 10.7 | 14.3 | 7.1 |
| | Magnesium stearate | 1.0 | 1.0 | 1.0 |
| | Flavourings | 4.1 | 4.1 | 4.1 |
| | Sweeteners | 0.7 | 0.7 | 0.7 |

### Examples 53-55

| | | **Ex 53 (% w/w)** | **Ex 54 (% w/w)** | **Ex 55 (% w/w)** |
|---|---|---|---|---|
| Granular composition: | | | | |
| | Ibuprofen | 28.6 | 28.6 | 28.6 |
| | Croscarmellose sodium | 5.2 | 5.2 | - |
| | Sodium starch glycolate | - | - | 5.2 |

| Extra-granular material: | | | | |
|---|---|---|---|---|
| | Xylitol | 37.4 | 35.4 | - |
| | Sorbitol | - | - | 35.4 |
| | Citric acid | 14.3 | 14.3 | 14.3 |
| | Sodium bicarbonate | 10.7 | 10.7 | 10.7 |
| | Magnesium stearate | 1.0 | 1.0 | 1.0 |
| | Flavourings | 2.1 | 4.1 | 4.1 |
| | Sweeteners | 0.7 | 0.7 | 0.7 |

### Examples 56-58

| | | **Ex 56 (% w/w)** | **Ex 57 (% w/w)** | **Ex 58 (% w/w)** |
|---|---|---|---|---|
| Granular composition: | | | | |
| | Ibuprofen | 28.6 | 28.6 | 28.6 |
| | Sodium starch glycolate | 4.3 | 5.2 | 5.2 |

| Extra-granular material: | | | | |
|---|---|---|---|---|
| | Xylitol | 25.6 | 42.6 | 37.4 |
| | Citric acid | 21.4 | 10.7 | 14.3 |
| | Sodium bicarbonate | 14.3 | 7.1 | 10.7 |
| | Magnesium stearate | 1.0 | 1.0 | 1.0 |
| | Flavourings | 4.1 | 4.1 | 2.1 |
| | Sweeteners | 0.7 | 0.7 | 0.7 |

### Examples 59-61

| | | **Ex 59 (% w/w)** | **Ex 60 (% w/w)** | **Ex 61 (% w/w)** |
|---|---|---|---|---|
| Granular composition: | | | | |
| | Ibuprofen | 14.3 | 11.8 | 14.3 |
| | Croscarmellose sodium | 5.1 | 3.5 | 5.2 |

| Extra-granular material: | | | | |
|---|---|---|---|---|
| | Xylitol | 49.8 | 50.5 | 56.9 |
| | Malic acid | 14.3 | 17.6 | 10.7 |
| | Sodium bicarbonate | 10.7 | 11.8 | 7.1 |
| | Magnesium stearate | 1.0 | 0.8 | 1.0 |
| | Flavourings | 4.1 | 3.4 | 4.1 |
| | Sweeteners | 0.7 | 0.6 | 0.7 |

### Examples 62-64

| | | **Ex 62 (% w/w)** | **Ex 63 (% w/w)** | **Ex 64 (% w/w)** |
|---|---|---|---|---|
| Granular composition: | | | | |
| | Ibuprofen | 13.6 | 14.3 | 11.8 |
| | Croscarmellose sodium | 4.9 | 5.1 | 3.5 |

| Extra-granular material: | | | | |
|---|---|---|---|---|
| | Xylitol | 54.2 | 49.8 | - |
| | Sorbitol | - | - | 50.5 |
| | Malic acid | 13.5 | 14.3 | 17.6 |
| | Sodium bicarbonate | 10.2 | 10.7 | 11.8 |
| | Magnesium stearate | 0.9 | 1.0 | 0.8 |
| | Flavourings | 2.0 | 4.1 | 3.4 |
| | Sweeteners | 0.7 | 0.7 | 0.6 |

### Examples 65-66

| | | **Ex 65 (% w/w)** | **Ex 66 (% w/w)** |
|---|---|---|---|
| Granular composition; | | | |
| | Ibuprofen | 14.3 | 13.5 |
| | Croscarmellose sodium | 5.2 | 5.0 |

| Extra-granular compositions | | | |
|---|---|---|---|
| | Xylitol | - | - |
| | Sorbitol | 56.9 | 54.2 |
| | Malic acid | 10.7 | 13.5 |
| | Sodium bicarbonate | 7.1 | 10.2 |
| | Magnesium stearate | 1.0 | 0.9 |
| | Flavourings | 4.1 | 2.0 |
| | Sweeteners | 0.7 | 0.7 |

### Example 67

The formulations of Examples 1 to 66 may be repeated except that the acid component of the extra-granular material instead forms part of the granular composition. In these examples, the acid component is added to the molten NSAID and mixed for 5-10 minutes until uniformly dispersed.

## Claims

1. A pharmaceutical formulation adapted to disintegrate in the mouth comprising a non-steroidal anti-inflammatory drug and an organic acid **characterised in that** the formulation comprises
(a) a granular composition comprising a plurality of solidified melt granules of said non-steroidal anti-inflammatory drug; and
(b) and 5-30% w/w of an organic acid.

2. A pharmaceutical formulation according to Claim 1, wherein the non-steroidal anti-inflammatory drug has a melting point in the range 20-300°C.

3. A formulation according to Claim 1 or Claim 2 **characterised in that** it comprises 1-50% by weight of said granular composition.

4. A formulation according to Claim 3 **characterised in that** it comprises 2-20% by weight of said granular composition.

5. A formulation according to any one of the preceding claims **characterised in that** it comprises a diluent.

6. A formulation according to Claim 5 **characterised in that** the diluent comprises an alkali metal salt, preferably in a ratio to said acid of 3:1 to 1:3 parts by weight.

7. A formulation according to any one of the preceding claims comprising said organic acid in an amount of 1-30% by weight.

8. A formulation according to Claim 7 **characterised in that** it comprises 10- . 22% by weight organic acid.

9. A formulation.according to any one of Claims 2 to 8 **characterised in that** the non-steroidal anti-inflammatory drug has a melting point in the range 40-200°C.

10. A formulation according to any one of Claims 5 to 9 **characterised in that** the diluent comprises at least one of xylitol, sorbitol, mannitol, fructose, sucrose, lactose, microcrystalline cellulose and dicalcium phosphate.

11. A formulation according to any one of the preceding claims **characterised in that** the formulation further comprises flavourings and/or sweeteners.

12. A formulation according to any one of the preceding claims **characterised in that** the formulation further comprises at least one of a gelling agent and/or a binder.

13. A formulation according to any one of the preceding claims, **characterised in that** the formulation comprises 5-20% by weight the von-steroidal anti-inflammatory drug.

14. A formulation according to any one of the preceding claims wherein the ratio of the organic acid to said non-steroidal anti-inflammatory drug is in the range 10:1 to 1:10 parts by weight.

15. A formulation according to any one of the preceding claims **characterised in that** it comprises an extra-granular composition comprising the organic acid.

16. A formulation according to any one of the preceding claims comprising an alkali metal salt base in an amount of 5-20% by weight.

17. A formulation according to any one of the preceding claims wherein the non-steroidal anti-inflammatory drug comprises at least one of ibuprofen, flurbiprofen, ketoprofen and naproxen or enantiomers thereof.

18. A formulation according to Claim 17 comprising racemic ibuprofen or S(+)-ibuprofen.

19. A formulation according to any one of the preceding claims, wherein said acid comprises at least one of citric acid, malic acid; tartaric acid, fumaric acid, ascorbic acid and adipic acid.

20. A formulation according to any one of Claims 16 to 19 wherein the base comprises at least one of sodium carbonate, sodium bicarbonate, potassium carbonate and potassium bicarbonate.

21. A formulation according to any one of the preceding claims further comprising a disintegrant.

22. A formulation according to Claim 21 wherein the granular composition comprises 1-50% by weight of the formulation of a disintegrant.

23. A formulation according to Claim 21 or 22 wherein the disintegrant comprises at least one of croscarmellose sodium and sodium starch glycollate.

24. A formulation according to any one of Claims 15 to 23, wherein the extra-granular composition comprises silicon dioxide.

25. A formulation according to any one of the preceding claims further comprising a surfactant.

26. A formulation according to any one of Claims 5 to 25, wherein the diluent is present in an amount of 30-80% by weight.

27. A formulation according to any one of the preceding claims comprising:
(I) a granular composition comprising:
(a) 85-95% w/w non-steroidal anti-inflammatory drug, and
(b) 5-15% w/w disintegrant
and
(II) an extra-granular composition comprising:
(c) 10-90% w/w diluent;
(d) 5-30% w/w acid;
(e) 5-30% w/w base, and
(f) 0-5% w/w surfactant.

28. A formulation according to any one of the preceding claims comprising:
(a) 5-25% w/w ibuprofen;
(b) 5-30% w/w acidic component;
(c) 0.1-5% w/w croscarmellose sodium;
(d) 5-20% w/w sodium bicarbonate; and
(e) 50-70% w/w diluent.

29. A formulation according to any one of Claims 1-28 in the form of a unit dose.

30. A formulation according to any one of Claims 1-29 in the form of a powder mixture or a tablet.

31. A formulation according to any one of Claims 1 to 30 for use in the treatment of pain and/or inflammation and/or fever.

32. A formulation according to Claim 31 for use in the treatment of coughs, colds, influenza, migraine, headache, rheumatic pain, muscular pain and neuralgia.

33. A chewable tablet adapted to disintegrate in the mouth and comprising:
(a) a granular composition comprising: a plurality of solidified melt granules of a non-steroidal anti-inflammatory drug; and
(b) an extra-granular composition comprising an organic acid
**characterised in that** said melt granules comprise a continuous phase of said non-steroidal anti-inflammatory drug.

34. A tablet according to Claim 33, wherein the non-steroidal anti-inflammatory drug has a melting point in the range 20-300°C.

35. A tablet according to Claim 33 or Claim 34 further comprising a disintegrant.

36. A process to prepare a formulation according to any one of Claims 1-32 **characterised by** the following steps :
(a) melting said non-steroidal anti-inflammatory drug optionally with one or more excipients;
(b) forming said mixture into solidified melt granules;
(c) combining said solidified melt granules with an organic acid to form a uniform mixture; and optionally
(d) forming into a unit dose.

37. A process according to Claim 36, wherein in step (b) the mixture is cooled to the solid state before being formed into granules.

38. A process according to either one of Claims 36 and 37 **characterised in that** the one or more excipients and solid drug are mixed uniformly in the solid state at the beginning of step (a).

39. A process according to any one of Claims 36-38 **characterised in that** said drug is melt-extruded in step (a).

40. A process according to any one of Claims 36-39 **characterised in that** the drug and acidic component are heated to a temperature in the range 80-130°C to melt said drug.

41. A process according to any one of Claims 36-40 **characterised in that** the drug is fully melted in step (a).

42. A process according to any one Claims 36-41 **characterised in that** in step (a) a disintegrant is combined with said drug in molten form.

43. A process according to any one of Claims 36-42 **characterised in** step (a) a surfactant is combined with said drug in molten form.

44. A process according to any one of Claims 36-43 **characterised in that** said organic acid forms part of an extra-granular composition which further comprises at least one of silicon dioxide, stearic acid or a salt thereof, a surfactant and a diluent.

45. A process according to any one of Claims 36-44 **characterised in that** said drug comprises ibuprofen.

46. The use of a formulation according to any one of Claims 1-30 in the manufacture of a medicament for the treatment of pain and/or inflammation and/or fever.

47. The use according to Claim 46 for the manufacture of a medicament for the treatment of coughs, colds, influenza, migraine, headache, rheumatic pain, muscular pain and neuralgia.

## Patentansprüche

1. Pharmazeutische Formulierung, die dazu geeignet ist, um sich im Mund zu zersetzen, umfassend einen nicht steroidalen Entzündungshemmer und eine organische Säure, **dadurch gekennzeichnet, dass** die Formulierung umfasst:
a) eine granuläre Zusammensetzung, umfassend eine Mehrzahl von erstarrten geschmolzenen Granulaten des nicht steroidalen Entzündungshemmers; und
b) 5 bis 30 Gew.-% einer organischen Säure.

2. Pharmazeutische Formulierung nach Anspruch 1, wobei der nicht steroidale Entzündungshemmer einen Schmelzpunkt im Bereich von 20 bis 300°C aufweist.

3. Formulierung nach Anspruch 1 oder 2, **dadurch gekennzeichnet, dass** sie 1 bis 50 Gew.-% der granulären Zusammensetzung umfasst.

4. Formulierung nach Anspruch 3, **dadurch gekennzeichnet, dass** sie 2 bis 20 Gew.-% der granulären Zusammensetzung umfasst.

5. Formulierung nach einem der vorherigen Ansprüche, **dadurch gekennzeichnet, dass** sie ein Verdünnungsmittel umfasst.

6. Formulierung nach Anspruch 5, **dadurch gekennzeichnet, dass** das Verdünnungsmittel ein Alkalimetallsalz umfasst, vorzugsweise in einem Verhältnis zur Säure von 3:1 bis 1:3 Teilen nach Gewicht.

7. Formulierung nach einem der vorherigen Ansprüche, umfassend die organische Säure in einer Menge von 1 bis 30 Gew.-%.

8. Formulierung nach Anspruch 7, **dadurch gekennzeichnet, dass** sie 10 bis 22 Gew.-% organische Säure umfasst.

9. Formulierung nach einem der Ansprüche 2 bis 8, **dadurch gekennzeichnet, dass** der nicht steroidale Entzündungshemmer einen Schmelzpunkt im Bereich von 40 bis 200°C aufweist.

10. Formulierung nach einem der Ansprüche 5 bis 9, **dadurch gekennzeichnet, dass** das Verdünnungsmittel zumindest eines von Xylit, Sorbit, Mannit, Fruktose, Sukrose, Laktose, mikrokristalliner Zellulose und Dicalciumphosphat umfasst.

11. Formulierung nach einem der vorherigen Ansprüche, **dadurch gekennzeichnet, dass** die Formulierung ferner Geschmacksstoffe und/oder Süßstoffe umfasst.

12. Formulierung nach einem der vorherigen Ansprüche, **dadurch gekennzeichnet, dass** die Formulierung ferner zumindest eines von Geliermittel und/oder Bindungsmittel umfasst.

13. Formulierung nach einem der vorherigen Ansprüche, **dadurch gekennzeichnet, dass** die Formulierung 5 bis 20 Gew.-% des nicht steroidalen Entzündungshemmers umfasst.

14. Formulierung nach einem der vorherigen Ansprüche, wobei das Verhältnis der organischen Säure zum nicht steroidalen Entzündungshemmer im Bereich von 10:1 bis 1:10 Teilen nach Gewicht liegt.

15. Formulierung nach einem der vorherigen Ansprüche, **dadurch gekennzeichnet, dass** sie eine extragranuläre Zusammensetzung, die die organische Säure umfasst, umfasst.

16. Formulierung nach einem der vorherigen Ansprüche, umfassend eine Alkalimetallsalzbase in einer Menge von 5 bis 20 Gew.-%.

17. Formulierung nach einem der vorherigen Ansprüche, wobei der nicht steroidale Entzündungshemmer zumindest eines von Ibuprofen, Flurbiprofen, Ketoprofen und Naproxen oder Enantiomeren davon umfasst.

18. Formulierung nach Anspruch 17, umfassend razemisches Ibuprofen oder S(+)-Ibuprofen.

19. Formulierung nach einem der vorherigen Ansprüche, wobei die Säure zumindest eine von Zitronensäure, Apfelsäure, Weinsäure, Fumarsäure, Ascorbinsäure und Adipinsäure umfasst.

20. Formulierung nach einem der Ansprüche 16 bis 19, wobei die Base zumindest eines von Natriumcarbonat, Natriumbicarbonat, Kaliumcarbonat und Kaliumbicarbonat umfasst.

21. Formulierung nach einem der vorherigen Ansprüche, ferner umfassend ein Sprengmittel.

22. Formulierung nach Anspruch 21, wobei die granuläre Zusammensetzung 1 bis 50 Gew.-% der Formulierung eines Sprengmittels umfasst.

23. Formulierung nach Anspruch 21 oder 22, wobei das Sprengmittel zumindest eines von Croscarmellose-Natrium und Natriumstärkeglykolat umfasst.

24. Formulierung nach einem der Ansprüche 15 bis 23, wobei die extragranuläre Zusammensetzung Siliciumdioxid umfasst.

25. Formulierung nach einem der vorherigen Ansprüche, ferner umfassend ein oberflächenaktives Mittel.

26. Formulierung nach einem der Ansprüche 5 bis 25, wobei das Verdünnungsmittel in einer Menge von 30 bis 80 Gew.-% vorliegt.

27. Formulierung nach einem der vorherigen Ansprüche, umfassend:
(I) eine granuläre Zusammensetzung, umfassend:
a) 85 bis 95 Gew.-% nicht steroidaler Entzündungshemmer, und
b) 5 bis 15 Gew.-% Sprengmittel
und
(II) eine extragranuläre Zusammensetzung, umfassend:
c) 10 bis 90 Gew.-% Verdünnungsmittel;
d) 5 bis 30 Gew.-% Säure;
e) 5 bis 30 Gew.-% Base, und
f) 0 bis 5 Gew.-% oberflächenaktives Mittel.

28. Formulierung nach einem der vorherigen Ansprüche, umfassend:
a) 5 bis 25 Gew.-% Ibuprofen;
b) 5 bis 30 Gew.-% saure Komponente;
c) 0,1 bis 5 Gew.-% Croscarmellose-Natrium;
d) 5 bis 20 Gew.-% Natriumbicarbonat; und
e) 50 bis 70 Gew.-% Verdünnungsmittel.

29. Formulierung nach einem der Ansprüche 1 bis 28 in Form einer Einheitsdosis.

30. Formulierung nach einem der Ansprüche 1 bis 29 in Form einer Pulvermischung oder Tablette.

31. Formulierung nach einem der Ansprüche 1 bis 30 zur Verwendung bei der Behandlung von Schmerzen und/oder Entzündung und/oder Fieber.

32. Formulierung nach Anspruch 31 zur Verwendung bei der Behandlung von Husten, Erkältung, Influenza, Migräne, Kopfschmerzen, Rheumaschmerzen, Muskelschmerzen und Neuralgie.

33. Kautablette, die geeignet ist, um sich im Mund zu zersetzen, umfassend:
a) eine granuläre Zusammensetzung, umfassend eine Mehrzahl von erstarrten geschmolzenen Granulaten eines nicht steroidalen Entzündungshemmers; und
b) eine extragranuläre Zusammensetzung, umfassend eine organische Säure; **dadurch gekennzeichnet, dass** die geschmolzenen Granulate eine kontinuierliche Phase des nicht steroidalen Entzündungshemmers umfassen.

34. Tablette nach Anspruch 33, wobei der nicht steroidale Entzündungshemmer einen Schmelzpunkt im Bereich von 20 bis 300°C aufweist.

35. Tablette nach Anspruch 33 oder 34, ferner umfassend ein Sprengmittel.

36. Verfahren zum Herstellen einer Formulierung nach einem der Ansprüche 1 bis 32, **gekennzeichnet durch** die folgenden Schritte:
a) Schmelzen des nicht steroidalen Entzündungshemmers optional mit einem oder mehreren Exzipienten;
b) Formen der Mischung zu erstarrten geschmolzenen Granulaten;
c) Kombinieren der erstarrten geschmolzenen Granulate mit einer organischen Säure, um eine einheitliche Mischung zu bilden; und optional
d) Formen in eine Einheitsdosis.

37. Verfahren nach Anspruch 36, wobei die Mischung in Schritt b) in den festen Zustand gekühlt wird, bevor sie zu Granulaten geformt wird.

38. Verfahren nach einem der Ansprüche 36 und 37, **dadurch gekennzeichnet, dass** der eine oder die mehreren Exzipienten und das feste Arzneimittel im festen Zustand zu Beginn von Schritt a) einheitlich vermischt werden.

39. Verfahren nach einem der Ansprüche 36 bis 38, **dadurch gekennzeichnet, dass** das Arzneimittel in Schritt a) schmelzextrudiert wird.

40. Verfahren nach einem der Ansprüche 36 bis 39, **dadurch gekennzeichnet, dass** das Arzneimittel und die saure Komponente auf eine Temperatur im Bereich von 80 bis 130°C erhitzt werden, um das Arzneimittel zu schmelzen.

41. Verfahren nach einem der Ansprüche 36 bis 40, **dadurch gekennzeichnet, dass** das Arzneimittel in Schritt a) zur Gänze geschmolzen wird.

42. Verfahren nach einem der Ansprüche 36 bis 41, **dadurch gekennzeichnet, dass** ein Sprengmittel in Schritt a) mit dem Arzneimittel in geschmolzener Form kombiniert wird.

43. Verfahren nach einem der Ansprüche 36 bis 42, **dadurch gekennzeichnet, dass** ein oberflächenaktives Mittel in Schritt a) mit dem Arzneimittel in geschmolzener Form kombiniert wird.

44. Verfahren nach einem der Ansprüche 36 bis 43, **dadurch gekennzeichnet, dass** die organische Säure Teil einer extragranulären Zusammensetzung ist, die ferner zumindest eines von Siliciumdioxid, Stearinsäure oder ein Salz davon, ein oberflächenaktives Mittel und Verdünnungsmittel umfasst.

45. Verfahren nach einen der Ansprüche 36 bis 44, **dadurch gekennzeichnet, dass** das Arzneimittel Ibuprofen umfasst.

46. Verwendung einer Formulierung nach einem der Ansprüche 1 bis 30 bei der Herstellung eines Medikaments zur Behandlung von Schmerzen und/oder Entzündung und/oder Fieber.

47. Verwendung nach Anspruch 46 zur Herstellung eines Medikaments zur Behandlung von Husten, Erkältung, Influenza, Migräne, Kopfschmerzen, Rheumaschmerzen, Muskelschmerzen und Neuralgie.

## Revendications

1. Formulation pharmaceutique conçue pour se désintégrer dans la bouche comprenant un anti-inflammatoire non stéroïdien et un acide organique, **caractérisée en ce que** la formulation comprend
a) une composition granulaire comprenant une pluralité de granulés sous la forme d'un liquide solidifié dudit anti-inflammatoire non stéroïdien ; et
b) et de 5 à 30 % en poids d'un acide organique.

2. Formulation pharmaceutique selon la revendication 1, dans laquelle l'anti-inflammatoire non stéroïdien a un point de fusion dans la plage de 20 à 300 °C.

3. Formulation selon la revendication 1 ou la revendication 2, **caractérisée en ce qu'**elle comprend de 1 à 50 % en poids de ladite composition granulaire.

4. Formulation selon la revendication 3, **caractérisée en ce qu'**elle comprend de 2 à 20 % en poids de ladite composition granulaire.

5. Formulation selon l'une quelconque des revendications précédentes, **caractérisée en ce qu'**elle comprend un diluant.

6. Formulation selon la revendication 5, **caractérisée en ce que** le diluant comprend un sel de métal alcalin, de préférence dans un rapport audit acide de 3/1 à 1/3 parties en poids.

7. Formulation selon l'une quelconque des revendications précédentes comprenant ledit acide organique en une quantité de 1 à 30 % en poids.

8. Formulation selon la revendication 7, **caractérisée en ce qu'**elle comprend de 10 à 22 % en poids d'acide organique.

9. Formulation selon l'une quelconque des revendications 2 à 8, **caractérisée en ce que** l'anti-inflammatoire non stéroïdien a un point de fusion dans la plage de 40 à 200 °C.

10. Formulation selon l'une quelconque des revendications 5 à 9, **caractérisée en ce que** le diluant comprend au moins l'un du xylitol, du sorbitol, du mannitol, du fructose, du sucrose, du lactose, de la cellulose microcristalline et du phosphate dicalcique.

11. Formulation selon l'une quelconque des revendications précédentes, **caractérisée en ce que** la formulation comprend en outre des aromatisants et/ou des édulcorants.

12. Formulation selon l'une quelconque des revendications précédentes, **caractérisée en ce que** la formulation comprend en outre au moins l'un d'un agent gélifiant et/ou d'un liant.

13. Formulation selon l'une quelconque des revendications précédentes, **caractérisée en ce que** la formulation comprend de 5 à 20 % en poids de l'anti-inflammatoire non stéroïdien.

14. Formulation selon l'une quelconque des revendications précédentes, dans laquelle le rapport de l'acide organique audit anti-inflammatoire non stéroïdien est dans la plage de 10/1 à 1/10 parties en poids.

15. Formulation selon l'une quelconque des revendications précédentes, **caractérisée en ce qu'**elle comprend une composition extra-granulaire comprenant l'acide organique.

16. Formulation selon l'une quelconque des revendications précédentes comprenant une base constituée d'un sel de métal alcalin en une quantité de 5 à 20 % en poids.

17. Formulation selon l'une quelconque des revendications précédentes dans laquelle l'anti-inflammatoire non stéroïdien comprend au moins l'un de l'ibuprofène, du flurbiprofène, du cétoprofène et du naproxène ou des énantiomères de ceux-ci.

18. Formulation selon la revendication 17 comprenant de l'ibuprofène racémique ou du S(+)-ibuprofène.

19. Formulation selon l'une quelconque des revendications précédentes, dans laquelle ledit acide comprend au moins l'un de l'acide citrique, l'acide malique, l'acide tartarique, l'acide fumarique, l'acide ascorbique et l'acide adipique.

20. Formulation selon l'une quelconque des revendications 16 à 19 dans laquelle la base comprend au moins l'un du carbonate de sodium, du bicarbonate de sodium, du carbonate de potassium et du bicarbonate de potassium.

21. Formulation selon l'une quelconque des revendications précédentes comprenant en outre un délitant.

22. Formulation selon la revendication 21 dans laquelle la composition granulaire comprend de 1 à 50 % en poids de la formulation d'un délitant.

23. Formulation selon la revendication 21 ou 22 dans laquelle le délitant comprend au moins l'un de la croscarmellose sodique et du glycolate d'amidon sodique.

24. Formulation selon l'une quelconque des revendications 15 à 23, dans laquelle la composition extra-granulaire comprend du dioxyde de silicium.

25. Formulation selon l'une quelconque des revendications précédentes comprenant en outre un tensioactif.

26. Formulation selon l'une quelconque des revendications 5 à 25, dans laquelle le diluant est présent en une quantité de 30 à 80 % en poids.

27. Formulation selon l'une quelconque des revendications précédentes comprenant :
(I) une composition granulaire comprenant :
(a) de 85 à 95 % en poids d'anti-inflammatoire non stéroïdien, et
(b) de 5 à 15 % en poids de délitant
et
(II) une composition extra-granulaire comprenant :
c) de 10 à 90 % en poids de diluant ;
d) de 5 à 30 % en poids d'acide ;
(e) de 5 à 30 % en poids de base, et
(f) de 0 à 5 % en poids de tensioactif.

28. Formulation selon l'une quelconque des revendications précédentes comprenant :
(a) de 5 à 25 % en poids d'ibuprofène ;
(b) de 5 à 30 % en poids de composant acide ;
(c) de 0,1 à 5 % en poids de croscarmellose sodique ;
(d) de 5 à 20 % en poids de bicarbonate de sodium ; et
(e) de 50 à 70 % en poids de diluant.

29. Formulation selon l'une quelconque des revendications 1 à 28 sous la forme d'une dose unitaire.

30. Formulation selon l'une quelconque des revendications 1 à 29 sous la forme d'une poudre mélangée ou d'un comprimé.

31. Formulation selon l'une quelconque des revendications 1 à 30 à utiliser dans le traitement de la douleur et/ou de l'inflammation et/ou de la fièvre.

32. Formulation selon la revendication 31 à utiliser dans le traitement de la toux, du rhume, de la grippe, de la migraine, des céphalées, des douleurs rhumatismales, des douleurs musculaires et de la névralgie.

33. Comprimé à mâcher conçu pour se désintégrer dans la bouche et comprenant :
a) une composition granulaire comprenant une pluralité de granulés sous la forme d'un liquide solidifié d'un anti-inflammatoire non stéroïdien ; et
b) une composition extra-granulaire comprenant un acide organique
**caractérisé en ce que** lesdits granulés formés à partir d'un liquide comprennent une phase continue dudit anti-inflammatoire non stéroïdien.

34. Comprimé selon la revendication 33, dans lequel l'anti-inflammatoire non stéroïdien a un point de fusion dans la plage de 20 à 300 °C.

35. Comprimé selon la revendication 33 ou la revendication 34 comprenant en outre un délitant.

36. Procédé de préparation d'une formulation selon l'une quelconque des revendications 1 à 32, **caractérisé par** les étapes suivantes :
(a) fusion dudit anti-inflammatoire non stéroïdien, facultativement avec un ou plusieurs excipients ;
(b) préparation de granulés sous la forme d'un liquide solidifié à partir dudit mélange ;
(c) combinaison desdits granulés sous la forme d'un liquide solidifié avec un acide organique pour former un mélange uniforme ; et facultativement
(d) mise sous la forme d'une dose unitaire.

37. Procédé selon la revendication 36, dans lequel, dans l'étape (b), le mélange est refroidi à l'état solide avant d'être mis sous la forme de granulés.

38. Procédé selon l'une ou l'autre des revendications 36 et 37, **caractérisé en ce que** le ou les excipients et le médicament solide sont mélangés uniformément à l'état solide au début de l'étape (a).

39. Procédé selon l'une quelconque des revendications 36 à 38, **caractérisé en ce que** ledit médicament est extrudé à l'état fondu dans l'étape (a).

40. Procédé selon l'une quelconque des revendications 36 à 39, **caractérisé en ce que** le médicament et le composant acide sont chauffés à une température dans la plage de 80 à 130 °C pour faire fondre ledit médicament.

41. Procédé selon l'une quelconque des revendications 36 à 40, **caractérisé en ce que** le médicament est entièrement fondu dans l'étape (a).

42. Procédé selon l'une quelconque des revendications 36 à 41, **caractérisé en ce que** dans l'étape (a), un délitant est combiné avec ledit médicament sous forme fondue.

43. Procédé selon l'une quelconque des revendications 36 à 42, **caractérisé en ce que** dans l'étape (a), un tensioactif est combiné avec ledit médicament sous forme fondue.

44. Procédé selon l'une quelconque des revendications 36 à 43, **caractérisé en ce que** ledit acide organique constitue une partie d'une composition extra-granulaire qui comprend en outre au moins l'un du dioxyde de silicium, de l'acide stéarique ou d'un sel de celui-ci, d'un tensioactif et d'un diluant.

45. Procédé selon l'une quelconque des revendications 36 à 44, **caractérisé en ce que** ledit médicament comprend de l'ibuprofène.

46. Utilisation d'une formulation selon l'une quelconque des revendications 1 à 30 dans la fabrication d'un médicament destiné au traitement de la douleur et/ou de l'inflammation et/ou de la fièvre.

47. Utilisation selon la revendication 46 pour la fabrication d'un médicament destiné au traitement de la toux, du rhume, de la grippe, de la migraine, des céphalées, des douleurs rhumatismales, des douleurs musculaires et de la névralgie.
